Europäisches Patentamt

European Patent Office

Office européen des brevets

(11) Veröffentlichungsnummer: **0 274 104**
**A2**

## EUROPÄISCHE PATENTANMELDUNG

(21) Anmeldenummer: 87118874.4

(22) Anmeldetag: 18.12.87

(51) Int. Cl.⁴: **A61K.7/48** , A61K 31/19

(30) Priorität: 22.12.86 US 944119

(43) Veröffentlichungstag der Anmeldung:
13.07.88 Patentblatt 88/28

(84) Benannte Vertragsstaaten:
AT BE CH DE ES FR GB GR IT LI LU NL SE

(71) Anmelder: F. HOFFMANN-LA ROCHE & CO.
Aktiengesellschaft

CH-4002 Basel(CH)

(72) Erfinder: Leyden, James Joseph
360 Grays Lane
Haverford Pennsylvania 19401(US)

(74) Vertreter: Lederer, Franz, Dr. et al
Patentanwält Dr. Franz Lederer Lucile
Grahnstrasse 22
D-8000 München 80(DE)

(54) 13-cis-Retinsäure zur Verwendung in Präparaten gegen alternde Haut.

(57) 13-cis-Retinsäure kann in topischen Präparaten zur Behandlung lichtgeschädigter Haut (Altershaut) dienen.

EP 0 274 104 A2

## 13-cis-Retinsäure zur Verwendung in Präparaten gegen alternde Haut

Menschliche Haut gliedert sich in drei grössere Abschnitte, eine dünne Epithel-Deckschicht, eine Mittelzone, die als Dermis bezeichnet wird und die aus Kollagen und Elastin-Proteinfasern besteht, und eine Unterschicht aus Fett. Mit der Alterung der Haut treten Veränderungen in allen drei Schichten ein, die klinisch sichtbar sein können. Mit der Desorganisation der Dermis treten Runzeln, Falten und Erschaffung auf und Kollagen-und Elastinfasern werden durch anomal elastisches Gewebe ersetzt. Dieser Vorgang ist als Elastosis bekannt und besonders deutlich in Gebieten, die durch ultraviolettes Licht geschädigt sind. Die Anwesenheit anomal elastischen Materials führt zur Beeinträchtigung der elastischen Eigenschaften der Haut, die Haut faltet sich und erschlafft und erhält ein runzliges Aussehen. Obschon die Haut geschädigtes elastisches Gewebe in einem gewissen Ausmass reparieren kann, nimmt diese Fähigkeit mit dem Alter ab. Daher geht seit langem der Wunsch nach einem Mittel, das die Fähigkeit der Haut, durch Elastosis verursachte Schäden zu reparieren, beschleunigt und die Haut stimuliert, normales Kollagen und/oder elastisches Gewebe zu produzieren.

Für das Studium der Elastosis der Haut gibt es ein geeignetes Tiermodell. Ultraviolett-Strahllendosen, vergleichbar mit denen, die auf Menschen einwirken, verursachen Elastosis, vergleiche Kligman et al J. Invest Derm. 78:181, 1982. Johnston et al. (J. Invest Derm. 82:587, 1984) haben weiterhin gezeigt, dass Ultraviolettstrahlung (UV-B), die so eingestellt ist, dass sie Sonneneinstrahlung simuliert, zu einer durch quantitative Messung der Aminosäure Desmosin nachweisbaren signifikanten Zunahme an Hautelastin führt. Die Elastinmenge ist proportional der Desmosinmenge, die durch Säurehydrolyse von Elastin freigesetzt werden kann, vergleiche Uitto et al. Laboratory Investigations 49:1216, 1973. Da Elastin durch Messen des Desmosingehaltes quantitativ bestimmt werden kann, kann diese Methode zum Nachweis der Wirkung von Verbindungen benutzt werden, die die Produktion von Elastin hemmen oder stimulieren. Infolgedessen bewirkt ein Mittel, das die Reparatur ultraviolett-induzierter Elastosis, die das Faltigwerden der Haut verursacht, eine Abnahme an Desmosin, was eine Abnahme an elastotischem Material anzeigt. Tatsächlich wurde mittels dieser Methode nachgewiesen, dass verschiedene Verbindungen, wie all-trans Retinsäure und verschiedene Lichtfiltermittel Stoffe sind, die die Reparatur von durch Elastosis verursachten Hautschäden fördern, vergleiche Kligman Connective Tissue Research, 1984 Vol. 12 pp 139 (1984); Kligman Journal of Invest. Dermatol. 81:98 (1983) und Kligman U.S. Patent No. 4,603,146 Juli 29, 1986.

Erfindungsgemäss wurde gefunden, dass 13-cis-Retinsäure bei topischer Verabreichung auf die Haut eines Patienten die mit Elastosis verbundenen Symptome aufhebt. Durch topische Anwendung von 13-cis-Retinsäure auf haut von Patienten, die durch Elastosis geschädigt ist, können die Effekte des Alterns, Faltigwerdens und Sonnenschäden rückgängig gemacht und eine Verbesserung im Erscheinungsbild der Haut herbeigeführt werden.

13-cis-Retinsäure ist topisch angewandt worden, um Akne zu behandeln, vergleiche Britisches Patent No. 1,335,867 und U.S. Patent No. 4,487,782. Die Anwendung dieser Verbindung als Mittel gegen Akne ist jedoch in keiner Weise mit der Fähigkeit verbunden, das Aussehen der Haut durch Bekämpfung von Symptomen, die mit Elastosis verbunden sind, wie Altern, Faltigwerden und Sonnenschäden zu verbessern. Durch topische Verabreichung der Verbindung wird eine Beschleunigung der Reparatur von Hautschäden bewirkt, so dass die Haut ein glatteres und jüngeres Aussehen erhält.

Wie bereits erwähnt, macht die Verbindung 13-cis-Retinsäure bei topischer Verabreichung auf die Patientenhaut die mit Elastosis verbundenen Symptome rückgängig und mässigt und verzögert so die durch Altern, Faltigwerden oder Sonne verursachten Hautschäden. Diese Schäden werden mit zunehmendem Alter des Patienten Ausgeprägter, da die Fähigkeit der Haut zur Selbstreparatur infolge des Alterns abnimmt. Durch topische Verabreichnung von 13-cis-Retinsäure auf die Haut des Patienten in einer Menge, die für die Rückgängigmachung der mit Elastosis verbundenen Symptome wirksam ist, wird eine Beschleunigung der Hautreparatur erreicht, wobei die Haut ein glatteres und jüngeres Aussehen erhält. Die 13-cis-Retinsäure sollte auf diejenigen Hautgebiete aufgebracht werden, die durch Elastosis beeinträchtigt sind oder deren Behandlung gewünscht wird. Die erfindungsgemässe Verwendung von 13-cis-Retinsäure wirkt dem Altern und dem Faltigwerden der Haut entgegen und intensiviert die Wiederherstellung sonnengeschädigter Haut.

Die 13-cis-Retinsäure kann erfindungsgemäss auf der menschlichen Haut in herkömmlichen topischen Zusammensetzungen angewandt werden. Diese Zusammensetzungen können verwendet werden, um 13-cis-Retinsäure aus die Körperhaut zu bringen, insbesondere auf das Gesicht, die Beine, die Arme und Hände. Zur Erzielung bester Resultate wird die 13-cis-Retinsäurebehandlung begonnen, wenn der Patient ein Alter von zwischen 40 und 55 Jahren hat, die Elastosis zu erscheinen beginnt und ausgeprägter wird. Danach können die Präparate dauernd angewandt werden, um die altersbedingten, mit Elastosis verbundenen Schäden zu reduzieren. Im allgemeinen ist es vorzuziehen, die Behandlung zu beginnen, wenn der Patient ein Alter von etwa 40 Jahren erreicht hat und die Behandlung lebenslang fortzusetzen, um die Auswirkungen der Elastosis zu vermindern und jeden Rückfall zu vermeiden.

Die 13-cis-Retinsäure kann erfindungsgemäss in jeder herkömmlichen geeigneten topischen Verabreichungsform, d.h. in Kombination mit jedem herkömmlichen Träger, der für die topische Verabreichung geeignet ist, angewandt werden. Die 13-cis-Retinsäure kann erfindungsgemäss infolgedessen in jeder geeigneten topischen Komposition, wie einer Créme, einer Salbe, einer Seife, einer Lösung, einer Lotion, einer Emulsion oder einem Shampoo angewandt werden. Im allgemeinen enthalten diese topischen Präparate zur Erzielung bester Resultate etwa 0,001% bis etwas 1% Gewichtsprozent 13-cis-Retinsäure, bezogen auf die gesamte Komposition, wobei Mengen von etwa 0,01 bis 1 Gewichtsprozent besonders bevorzugt sind. Gewünschtenfalls können je nach der Art und dem Ausmass der Elastosis höhere Konzentrationen angewandt werden.

Bei der Herstellung dieser Kompositionen können alle konventionellen, nicht toxischen, dermatologisch anwendbaren Trägerstoffe, in denen 13-cis-Retinsäure stabil ist verwendet werden. Bevorzugt für den erfindungsgemässen Zweck sind herkömmliche kosmetische Präparate, die einen weiteren kosmetisch wirksamen Inhaltsstoff enthalten, der bei topischer Verabreichung auf die menschliche Haut einen kosmetischen Effekt ausübt. Solche Stoffe sind Befeuchtungsmittel, oberflächenaktive Stoffe, Weichmacher, Farbstoffe, Konditioner, bakterizide Mittel, Adstringentien und Detergentien.

Die erfindungsgemässen Präparate können gewünschtenfalls geeignete Lichtschutzfilter enthalten. Jedes herkömmliche Lichtschutzmittel kann für den erfindungsgemässen Zweck verwendet werden. Diese topischen 13-cis-Retinsäure-Präparate können herkömmliche Excipientien und Zusatzstoffe enthalten, die üblicherweise bei der Herstellung topischer Präparate angewandt werden. Beispiele solcher Stoffe sind Konservierungsmittel, Verdicker und Parfüms. Zusätzlich können herkömmliche Antioxidantien wie butyliertes Hydroxyanisol, Ascorbylpalmitat, Propylgallat, Zitronensäure, butyliertes Hydroxytoluol oder Ethoxyquin diesen Präparaten einverleibt werden. Weiterhin können die topischen Präparate Trägerstoffe enthalten, die im allgemeinen in solchen Präparaten verwendet werden. Die Präparate können übliche Verdickungsmittel, Emulgatoren und Viskositätsstabilisatoren sowie Geschmacksstoffe, Farbstoffe und Parfüms enthalten. Die 13-cis-Retinsäure enthaltenden topischen Präparate werden vorzugsweise mindestens einmal täglich oder mindestens zwei-bis dreimal wöchentlich auf die Haut aufgebracht. Um die Elastosis rückgängig zu machen und der Haut ein glattes und jüngeres Aussehen zu verleihen sollen die topischen Präparate vorzugsweise für die Dauer von mindestens 5 Monate aufgebracht werden. Danach sollten die 13-cis-Retinsäurepräparate weiter angewandt werden, um den Effekt der jüngeren und glatteren Haut aufrechtzuerhalten. Die Präparate können entsprechend den Bedürfnissen des Patienten nach Weisung des Arztes angewandt werden. Jedenfalls wird das Anwendungsschema für die Präparate vom Alter, dem Gewicht und dem Zustand der Haut des betreffenden Patienten abhängen.

Die nachstehenden Beispiele erläutern die Erfindung weiter, ohne sie zu beschränken.


<u>Beispiel 1</u>

<u>Behebung UV-B-verursachter Hautschäden bei der haarlosen Maus durch 13-cis-Retinsäure</u>

Haarlose Mäuse (HRS/J Stamm, Jackson Labs, zu Beginn des Experiments 5-7 Wochen alt) wurden dreimal wöchentlich mit einer Anordnung von 8 Westinghouse Bestrahlungslampen (FS40) die etwa 20 cm oberhalb der Tiere plaziert waren, bestrahlt. Die Strahlungsdosis wurde durch ein handelsübliches phototherapeutisches Kontrollgerät kontrolliert. Die UV-B Dosis wurde so gewählt, dass die Dosierung selten $0,06J/cm^2$ überschritt, ein minimales Erythem, jedoch keine Verbrennungen oder Narben verursachte. Nach einer Gesamtdosis von etwa $3,5J/cm^2$ ergab sich eine durch histologischen Befund gesicherte signifikante Elastosis, die durch Messungen des Elastins mittels Radioimmunoassay für Desmosin in der gesamten Haut bestätigt wurde. Der Desmosingehalt erhöhte sich um das zwei-bis dreifache nach $3,5J/cm^2$ UV-B Bestrahlung. Um die Hautschäden zu beheben, wurde die UV-Bestrahlung unterbrochen und Gruppen der Tiere wurden separat dreimal wöchentlich mit verschiedenen Dosen Verschiedener Konzentrationen von 13-cis-Retinsäure und all-trans-Retinsäure, in Aceton gelöst, behandelt. Die Lösungen wurden jede Woche in Konzentrationen von 25,50 und 100 µg in 100 µl Aceton frisch hergestellt und topisch auf ein Gebiet von etwa $10 cm^2$ auf den Rücken der Tiere aufgebracht. Eine Kontrollgruppe wurde nur mit Aceton behandelt.

Nach 10 Behandlungswochen wurden die Tiere getötet, Hautpräparate angefertigt und durch Luna-Färbung des Elastins das Ausmass der Wiederherstellung quantitativ gemessen. Bei diesem Versuchsmodell wurde die Wiederherstellung der Haut definiert als das Aussehen einer normalen Dermis, die sich von der Epidermis bis auf die Schicht von komprimiertem Elastin erstreckt. Das Ausmass der Wiederherstellung war durch die Breite dieser Zone gegeben. Das Gebiet der Zone auf einer Standardlänge des histologischen Schnitts wurde gemessen und die Resultate als Gesamtfläche in mm² pro 20 mikroskopische Felder ausgedrückt. Die Resultate sind in Tabelle I zusammengestellt.

## Tabelle I

### 13-cis-Retinsäure

| Gruppe | N | Gesamtfläche in der Reparaturzone |
|---|---|---|
| Kontrolle | 5 | $0,0148 \pm 0,0047$ |
| 25 µg | 3 | $0,0240 \pm 0,0107$ |
| 50 µg | 6 | $0,0288 \pm 0,0053$ |
| 100 µg | 7 | $0,0451 \pm 0,0096$* |

* $P < 0,05$ gegenüber Kontrolle

### all-trans Retinsäure

| Gruppe | N | Gesamtfläche in der Reparaturzone |
|---|---|---|
| Kontrolle | 5 | $0,0094 \pm 0,0040$ |
| 2.5 µg | 5 | $0,0117 \pm 0,0043$ |
| 10 µg | 3 | $0,0110 \pm 0,0090$ |
| 25 µg | 5 | $0,0226 \pm 0,0113$ |

Bei all trans Retinsäure betrug die maximal tolerierte Dosis 25 µg.

Es wurde beobachtet, dass mit 13-cis-Retinsäure eine Wiederherstellung der Haut erhalten werden konnte, die etwa 3,5 Mal derjenigen des Kontrollwertes betrug, wobei mit all trans Vitamin A Säure das maximale Ausmass der Wiederherstellung nur etwa das 2,5-fache des Kontrollwertes betrug. "N" bedeutet die Anzahl der Mäuse in einer Gruppe.

### Beispiel 2

Wirkung von 13-cis-Retinsäure auf durch UV-B-Bestrahlung verursachte Runzeln bei der haarlosen Maus

Des wurde gefunden, dass Dosen von UV-B-Strahlung, die bei der haarlosen Maus Hautschäden verursachen, das Auftreten von Runzeln auf der der Strahlung ausgesetzten Haut verursacht. Von den bestrahlten Gebieten wurden Hautreplikate mit Hilfe einer in der Zahntechnik verwendeten Flüssigkeit angefertigt, um die Hautoberfläche einer visuellen Prüfung zugänglich zu machen. Bei diesen Replikaten erscheinen Runzeln als Vertiefungen, die bei Bestrahlung mit Licht in einem flachen Winkel Schatten werfen. Charakteristisch für das Erscheinungsbild der Runzeln war das Auftreten von Rillen in einer regelmässigen Anordnung mit 2 mm Abstand. Das Ausmass dieser Fältelung wurde unter Verwendung dieses Erscheinungsbilds visuell bestimmt. Es wurde beobachtet, dass 13-cis-Retinsäure eine dosisabhängige Glättung der Runzeln verursachte, die mit der Höchstdosis von 100 µg gänzlich vollständig war. Die Resultate sind in der nachstehenden Tabelle II zusammengestellt.

## Tabelle II

Glättung von UV-B-verursachten Runzeln durch topisch verabreichte 13-cis-Retinsäure.

| Gruppe | N | Fältelungsindex |
|---|---|---|
| Kontrolle | 10 | $2{,}30 \pm 0{,}21$ |
| 10 µg | 9 | $1{,}80 \pm 0{,}10$ |
| 30 µg | 9 | $1{,}20 \pm 0{,}10$ ** |
| 100 µg | 7 | $1{,}06 \pm 0{,}06$ *** |

** $P < 0{,}001$  *** $P < 0{,}001$ gegenüber Kontrollwert

## Beispiel 3

### Topisches Gel

| Komponente | Menge |
|---|---|
| 13-cis-Retinsäure | 0,0525 Gewichtsteile |
| Hydroxypropylcellulose | 3,0000 Gewichtsteile |
| Aethanol | 96,8975 Gewichtsteile |
| Butyliertes Hydroxytoluol | 0,0500 Gewichtsteile |

## Beispiel 4
### Crème

| | Gewichtsprozent |
|---|---|
| 13-cis-Retinsäure | 0,005 - 0,5 |
| Lexemul AR - [Gemisch von Glycerylstearatstearamidoäthyl] diäthylamin | 15,0 |
| Cetylesterwax (Liponat SPS) | 2,5 |
| Stearinsäure | 2,5 |
| Isopropylmyristat | 5,0 |
| Butyliertes Hydroxyanisol | 0,01 |
| Myrj 52 [Polyoxyäthylen(40)stearat] | 4,0 |
| Keltrol F [Xanthingummi] | 0,5 |
| Propylenglycol | 5,0 |
| Benzoesäure | 0,2 |
| Wasser q.s. | 100,0 |

## Beispiel 5
### Crème

| | Gewichtsprozent |
|---|---|
| 13-cis-retinsäure | 0,005 - 0,5 |
| Stearylalcohol, | 10,0 |
| Cetylalkohol, | 2,0 |
| Stearinsäure | 2,0 |
| Isopropylpalmitat | 5,0 |
| Myrj 52 | 4,0 |
| Petrolatum, | 5,0 |
| Bienenwax, | 2,0 |
| Butyliertes Hydroxyanisol | 0,05 |

| | |
|---|---|
| Butyliertes Hydroxytoluol | 0,05 |
| Methylparaben | 0,2 |
| Propylparaben | 0,05 |
| Propylenglycol, | 12,0 |
| Dinatriumsequestren | 0,01 |
| Destilliertes Wasser q.s. | |

## Beispiel 6
### Gel

| | Gewichtsprozent |
|---|---|
| 13-cis-Retinsäure | 0,005 - 0,5 |
| Brij 99 | 10 |
| Klucel (Hydroxypropylcellulose) | 2,5 |
| Aethanol q.s. | 100,0 |

## Beispiel 7
### Gel

| | Gewichtsprozent |
|---|---|
| 13-cis-Retinsäure | 0,005 - 0,3 |
| Aethanol | 80,0 |
| Carbopol 940 (Carboxyvinylpolymer) | 1,0 |
| Diisopropanolamin | 1,7 |
| Sorbo (70% Sorbit-Lösung) | 5,0 |
| Destilliertes Wasser q.s. | 100,0 |

## Beispiel 8
### Lotion

| | Gewichtsprozent |
|---|---|
| 13-cis-Retinsäure | 0,005 - 0,5 |
| Stearinsäure | 5,0 |
| Isopropylpalmitat | 2,0 |
| Arlacel 165 (Gemisch von Glyzerinmonostearat und Polyoxyäthylen) | 5,0 |
| Butyliertes Hydroxyanisol | 0,05 |
| Butyliertes Hydroxytoluol | 0,05 |
| Methylparaben | 0,2 |
| Propylparaben | 0,05 |
| Sorbo (70% Sorbit-Lösung) | 20,0 |
| Carbopol 961 (Ammoniumsalz von polymerer Acrylsäure) | 0,1 |
| Destilliertes Wasser q.s. | 100 |

## Beispiel 9
### Lösung

| | |
|---|---|
| 13-cis-Retinsäure | 0,5gm |
| Isopropylmyristat q.s. | 100 ml |

7

## Beispiel 10

| Bestandteile | Gewichtsprozent |
|---|---|
| 13-cis-Retinsäure | 0,05% |
| Butyliertes Hydroxytoluol | 0,05% |
| Klucel | 3,00% |
| Aethanol (absolut) q.s. | 100ml |

**Ansprüche**

1. 13-cis-Retinsäure zur Anwendung bei der topischen Behandlung von Zuständen, die mit Hautelastosis verbunden sind.

2. 13-cis-Retinsäure zur Anwendung gemäss Anspruch 1, in topischen Präparaten, die mindestens 0,001 Gewichtsprozent 13-cis-Retinsäure und einen inerten, dermatologisch annehmbaren Träger enthalten.

3. 13-cis-Retinsäure zur Anwendung gemäss Anspruch 2 in Kompositionen, die 13-cis-Retinsäure in Mengen von etwa 0,01 bis etwa 1 Gewichtsprozent enthalten.

4. 13-cis-Retinsäure zur Anwendung gemäss Anspruch 2 oder 3, wobei das Präparat einen weiteren, kosmetisch wirksamen Inhaltsstoff enthält.

5. Verwendung von 13-cis-Retinsäure bei der Herstellung von topischen Präparaten zur Behandlung von Zuständen die mit Hautelastosis verbunden sind.

6. Verwendung gemäss Anspruch 5, wobei die topischen Präparate mindestens 0,001 Gewichtsprozent 13-cis-Retinsäure und einen inerten dermatologisch annehmbaren Träger enthalten.

7. Verwendung gemäss Anspruch 6, worin das topische Präparat 13-cis-Retinsäure in Mengen von etwa 0,01% bis etwa 1 Gewichtsprozent enthält.

8. Verwendung gemäss Anspruch 7, wobei das Präparat einen weiteren kosmetisch wirksamen Inhaltsstoff enthält.